(19) Europäisches Patentamt
European Patent Office
Office européen des brevets

(11) Veröffentlichungsnummer: **0 058 347**
**B1**

(12) # EUROPÄISCHE PATENTSCHRIFT

(45) Veröffentlichungstag der Patentschrift: **12.09.84**

(51) Int. Cl.³: **C 07 D 239/26**, A 01 N 43/54

(21) Anmeldenummer: **82100776.2**

(22) Anmeldetag: **04.02.82**

(54) **Benzyl-pyrimidinylalkyl-ether, Verfahren zu ihrer Herstellung, ihre Verwendung als Pflanzenwachstumsregulatoren und Fungizide sowie Zwischenprodukte und deren Herstellung.**

(30) Priorität: **14.02.81 DE 3105374**

(43) Veröffentlichungstag der Anmeldung: **25.08.82 Patentblatt 82/34**

(45) Bekanntmachung des Hinweises auf die Patenterteilung: **12.09.84 Patentblatt 84/37**

(84) Benannte Vertragsstaaten: **AT BE CH DE FR GB IT LI NL**

(56) Entgegenhaltungen:
**EP - A - 0 001 399**

**Chemical Abstracts 74, 112.010n (1971)**

(73) Patentinhaber: **BAYER AG, Konzernverwaltung RP Patentabteilung, D-5090 Leverkusen 1 Bayerwerk (DE)**

(72) Erfinder: **Holmwood, Graham, Dr., Katernbergerstrasse 184, D-5600 Wuppertal 1 (DE)**
Erfinder: **Lürssen, Klaus, Dr., August-Kierspel-Strasse 89, D-5060 Bergisch Gladbach 2 (DE)**
Erfinder: **Frohberger, Paul-Ernst, Dr., Willi-Baumeister-Strasse 5, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 058 347 B1

## Beschreibung

Die vorliegende Erfindung betrifft Benzyl-pyrimidinylalkyl-ether, ein Verfahren zu ihrer Herstellung sowie ihre Verwendung als Pflanzenwachstumsregulatoren und Fungizide. Ferner betrifft die Erfindung neue Pyrimidinyl-carbinole, die als Zwischenprodukte zur Herstellung von Benzyl-pyrimidinylalkyl-ethern dienen, sowie ein Verfahren zur Herstellung der Pyrimidinyl-carbinole.

Es ist bereits bekannt geworden, dass Hydroxy-pyrimidin-Derivate, wie z.B. 2-Chlorphenyl-4-fluorphenyl-pyrimidin-5-yl-methanol oder 4-Fluorphenyl-phenyl-pyrimidin-5-yl-methanol, gute pestizide Eigenschaften besitzen (vergleiche die US-Patente Nrn. 3818009, 3868244, 3869456 und 3887708).

Ausserdem ist bereits bekannt geworden, dass 3-substituierte Pyridin-Derivate, wie z.B. 1-(2,4-Dichlorphenoxy)-2-phenyl-1-pyridin-3-yl-2-ethanon oder 1-(4-Chlor-2-methyl-phenoxy)-2-phenyl-1-pyridin-3-yl-ethanon, gute fungizide Eigenschaften besitzen (vergleiche DE-OS Nr. 2909287).

Die Wirkung all dieser Verbindungen ist jedoch, insbesondere bei niedrigen Aufwandmengen und -konzentrationen, nicht immer voll befriedigend.

Es wurden neue Benzyl-pyrimidinylalkyl-ether der Formel

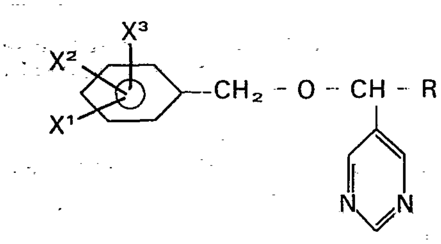 (I)

in welcher

R für Alkyl mit 1 bis 8 Kohlenstoffatomen, Cycloalkyl mit 3 bis 7 Kohlenstoffatomen oder für gegebenenfalls durch Halogen substituiertes Phenyl steht,

$X^1$ für Wasserstoff, Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen oder für Phenyl steht,

$X^2$ für Wasserstoff oder Halogen steht, und

$X^3$ für Wasserstoff oder Halogen steht,
sowie deren Säureadditionssalze und Metallsalz-Komplexe gefunden.

Die Verbindungen der Formel (I) besitzen ein asymmetrisches Kohlenstoffatom; sie können deshalb in den beiden optischen Isomeren oder als Racemat vorliegen. Sämtliche Isomeren werden erfindungsgemäss beansprucht.

Weiterhin wurde gefunden, dass man die Benzyl-pyrimidinylalkyl-ether der Formel (I), deren Säureadditionssalze und Metallsalz-Komplexe erhält, wenn man Pyrimidinyl-carbinole der Formel

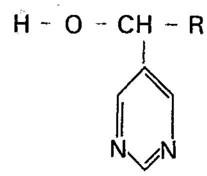 (II)

in welcher
R die oben angegebene Bedeutung hat,
mit Benzylhalogeniden der Formel

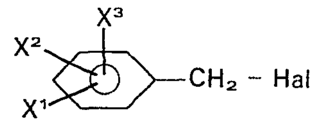 (III)

in welcher
$X^1$, $X^2$ und $X^3$ die oben angegebene Bedeutung haben und
Hal für Halogen steht,
in Gegenwart eines Lösungsmittels und gegebenenfalls in Gegenwart einer starken Base bzw. gegebenenfalls in Gegenwart eines Säurebindemittels umsetzt und gegebenenfalls eine Säure oder ein Metallsalz addiert.

Schliesslich wurde gefunden, dass die neuen Benzyl-pyrimidinylalkyl-ether der Formel (I) sowie deren Säureadditionssalze und Metallsalz-Komplexe starke pflanzenwachstumsregulierende und fungizide Eigenschaften aufweisen.

Überraschenderweise zeigen die erfindungsgemässen Verbindungen der Formel (I) eine bessere wachstumsregulierende Wirkung als die aus dem Stand der Technik bekannten Hydroxypyrimidin-Derivate sowie eine bessere fungizide Wirkung als die ebenfalls bekannten 3-substituierten Pyridin-Derivate. Die erfindungsgemässen Wirkstoffe stellen somit eine Bereicherung der Technik dar.

Die erfindungsgemässen Benzyl-pyrimidinylalkyl-ether sind durch die Formel (I) allgemein definiert.

Besonders bevorzugt sind diejenigen Verbindungen der Formel (I), in denen

R für Isopropyl, Isobutyl, tert.-Butyl, n-Pentyl, n-Hexyl, Cyclopentyl und Cyclohexyl sowie für gegebenenfalls durch Fluor, Chlor substituiertes Phenyl steht;

$X^1$ für Wasserstoff, Fluor, Chlor, Methyl, tert.-Butyl, Methoxy, Trifluormethyl, Trifluormethoxy, oder für Phenyl steht;

$X^2$ für Wasserstoff, Fluor, Chlor steht und

$X^3$ für Wasserstoff, Fluor, Chlor steht.

Im einzelnen seien ausser den bei den Herstellungsbeispielen genannten Verbindungen die folgenden Verbindungen der allgemeinen Formel (I) genannt:

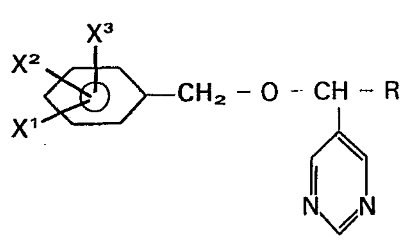 (I)

| $X^1$ | $X^2$ | $X^3$ | R |
|---|---|---|---|
| 4-Cl | H | H | 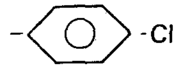 |
| 4-F | H | H | 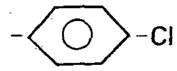 |
| 4-Cl | 2-Cl | H | 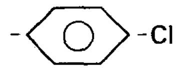 |
| 2-Cl | 6-F | H | 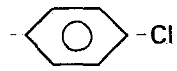 |
| 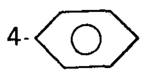 | H | H | 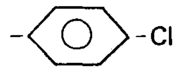 |
| 4-$CF_3$ | H | H | 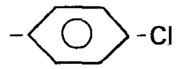 |
| 3-Cl | 4-Cl | H | 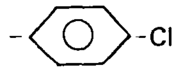 |
| 4-$OCH_3$ | H | H | 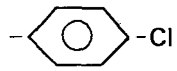 |
| 4-$OCF_3$ | H | H | 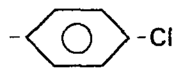 |
| 4-Cl | H | H | $-(CH_2)_5-CH_3$ |
| 4-F | H | H | $-(CH_2)_5-CH_3$ |
| 2-Cl | 4-Cl | H | $-(CH_2)_5-CH_3$ |
| 2-Cl | 6-F | H | $-(CH_2)_5-CH_3$ |
| 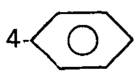 | H | H | $-(CH_2)_5-CH_3$ |
| 4-$CF_3$ | H | H | $-(CH_2)_5-CH_3$ |
| 3-Cl | H-Cl | H | $-(CH_2)_5-CH_3$ |
| 4-$OCH_3$ | H | H | $-(CH_2)_5-CH_3$ |
| 4-$OCF_3$ | H | H | $-(CH_2)_5-CH_3$ |

Verwendet man beispielsweise 2,2-Dimethyl-1-hydroxy-1-(pyrimidin-5-yl)-propan und 4-Chlorbenzylchlorid als Ausgangsstoffe und Natriumhydrid als Base, so kann der Reaktionsablauf durch das folgende Formelschema wiedergegeben werden:

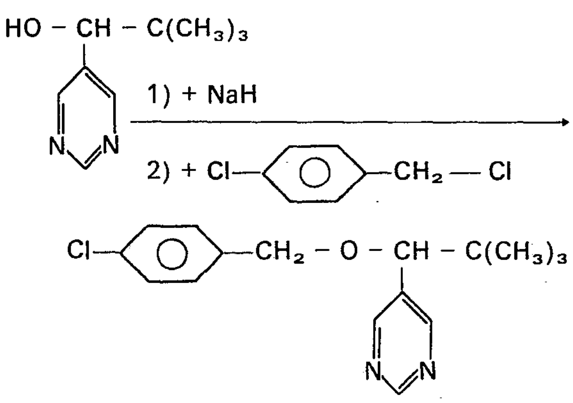


Die bei der Durchführung des erfindungsgemässen Verfahrens als Ausgangsstoffe zu verwendenden Pyrimidinyl-carbinole sind durch die Formel (II) allgemein definiert. In dieser Formel steht R vorzugsweise für diejenigen Reste, die bereits im Zusammenhang mit der Beschreibung der erfindungsgemässen Stoffe der Formel (I) für diesen Substituenten als besonders bevorzugt genannt wurden.

Die Pyrimidinyl-carbinole der Formel (II) sind teilweise bekannt [vgl. „Chemical Abstracts", *74*, 112010 n (1971)].

Die Pyrimidinyl-carbinole der Formel

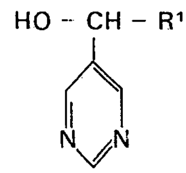 (IIa)

in welcher

$R^1$ für Isopropyl, Isobutyl, tert.-Butyl, n-Pentyl, n-Hexyl, Cyclopentyl, Cyclohexyl oder für gegebenenfalls durch Chlor substituiertes Phenyl steht, sind neu. Sie können jedoch in bekannter Art und Weise erhalten werden, indem man Pyrimidinhalogenide der Formel

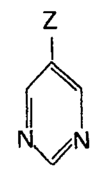 (IV)

in welcher

Z für Halogen, insbesondere Chlor oder Brom, steht, mit Aldehyden der Formel

$$O = CH-R^1 \quad (V)$$

in welcher

$R^1$ die oben angegebene Bedeutung hat,

in Gegenwart eines Verdünnungsmittels und in Gegenwart einer alkalimetall-organischen Verbindung umsetzt.

Die übrigen Pyrimidinyl-carbinole der Formel (II) lassen sich in analoger Weise herstellen.

Als Verdünnungsmittel kommen für die Herstellung der Pyrimidinyl-carbinole der Formel (IIa) vorzugsweise inerte organische Lösungsmittel in Frage. Hierzu gehören vorzugsweise solche, die einen tiefen Festpunkt besitzen, wie besonders Ether, wie Diethylether oder Tetrahydrofuran. Vorzugsweise arbeitet man mit Mischungen aus diesen beiden Ethern.

Die Herstellung der Verbindungen der Formel (IIa) erfolgt in Gegenwart einer alkalimetall-organischen Verbindung. Dazu werden vorzugsweise Alkalimetall-alkyle, wie insbesondere n-Butyl-lithium, eingesetzt; es können auch Alkalimetall-aryle, wie Phenyl-lithium, Verwendung finden.

Die Reaktionstemperaturen können bei der Durchführung dieses Verfahrens in einem bestimmten Bereich variiert werden. Im allgemeinen arbeitet man zwischen −150 und −50° C, vorzugsweise zwischen −120 und −80° C.

Dieses Verfahren wird vorzugsweise unter Inertgas, wie insbesondere Stickstoff oder Argon, durchgeführt.

Bei der Durchführung dieses Verfahrens setzt man auf 1 mol Pyrimidin-halogenid der Formel (IV) vorzugsweise 1 bis 2 mol Aldehyd der Formel (V) ein. Die alkalimetall-organische Verbindung wird vorteilhaft im Überschuss von 5 bis 75 Molprozent, vorzugsweise von 10 bis 50 Molprozent verwendet.

Die Isolierung der Verbindungen der Formel (IIa) erfolgt in der Weise, dass das bei der Reaktion zunächst gebildete Alkalialkanolat (z.B. Lithium-alkanolat) hydrolysiert wird, wie z.B. mit gesättigter Ammoniumchloridlösung oder mit Wasser. Die weitere Aufarbeitung erfolgt dann in üblicher Weise.

Die Pyrimidinyl-carbinole der Formel (II) stellen allgemein interessante Zwischenprodukte dar, wie z.B. zur Herstellung der erfindungsgemässen Verbindung der Formel (I).

Die Pyrimidin-halogenide der Formel (IV) und die Aldehyde der Formel (V) sind allgemein bekannte Verbindungen der organischen Chemie.

Die ausserdem bei der Durchführung des erfindungsgemässen Verfahrens als Ausgangsstoffe zu verwendenden Benzylhalogenide sind durch die Formel (III) allgemein definiert. In dieser Formel steht $X^1$, $X^2$ und $X^3$ vorzugsweise für diejenigen Reste, die bereits im Zusammenhang mit der Beschreibung der erfindungsgemässen Stoffe der Formel (I) für diese Substituenten als besonders bevorzugt genannt wurden. Hal steht vorzugsweise für Chlor oder Brom.

Die Benzylhalogenide der Formel (III) sind allgemein bekannte Verbindungen der organischen Chemie.

Als Lösungsmittel kommen für das erfindungsgemässe Verfahren vorzugsweise inerte organische Lösungsmittel in Frage. Hierzu gehören vorzugsweise Ether, wie Diethylether oder Dioxan; aromatische Kohlenwasserstoffe, wie Toluol und Benzol; Dimethylsulfoxid und Dimethylformamid.

Die Reaktionstemperaturen können bei der Durchführung des erfindungsgemässen Verfahrens in einem grösseren Bereich variiert werden. Im allgemeinen arbeitet man zwischen 0 und 100° C, vorzugsweise zwischen 20 und 80° C.

Das erfindungsgemässe Verfahren kann gegebenenfalls in Gegenwart einer starken Base durchgeführt werden. Hierzu gehören vorzugsweise Alkalimetall-hydride, Alkalimetall-amide und Alkalimetall-alkoholate, wie z.B. Natriumhydrid, Natriumamid und Kalium-tert.-butylat.

Das erfindungsgemässe Verfahren kann gegebenenfalls in Gegenwart von Säurebindern durchgeführt werden. Hierzu gehören organische Basen, vorzugsweise tertiäre Amine, sowie anorganische Basen, wie z.B. Alkalihydroxide.

Bei der Durchführung des erfindungsgemässen Verfahrens setzt man vorzugsweise auf 1 mol der Verbindungen der Formel (II) 1 bis 3 mol Benzylhalogenid der Formel (III) ein. Die Isolierung der Endprodukte der Formel (I) erfolgt in üblicher Weise.

In einer bevorzugten Ausführungsform wird zweckmässigerweise so verfahren, dass man das Pyrimidinyl-carbinol der Formel (II) in einem geeigneten inerten organischen Lösungsmittel mittels Alkalimetall-hydrid oder -amid in das Alkanolat überführt und letzteres ohne Isolierung sofort mit einem Benzylhalogenid der Formel (III) umsetzt, wobei unter Austritt von Alkalihalogenid die erfindungsgemässen Verbindungen der Formel (I) in einem Arbeitsgang erhalten werden.

Nach einer weiteren bevorzugten Ausführungsform wird die erfindungsgemässe Umsetzung in einem Zweiphasensystem vorgenommen, wie beispielsweise wässerige Natron- oder Kalilauge/Toluol oder Methylenchlorid, unter Zusatz von 0,01-1 mol eines Phasen-Transfer-Katalysators, wie beispielsweise einer Ammonium- oder Phosphoniumverbindung, beispielsweise seien Tetrabutylammoniumbromid, Benzyldodecyl-dimethylammoniumchlorid (Zephirol) und Triethylbenzylammoniumchlorid genannt.

Zur Herstellung von Säureadditionssalzen der Verbindungen der Formel (I) kommen vorzugsweise folgende Säuren in Frage: Die Halogenwasserstoffsäuren, wie zum Beispiel Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie zum Beispiel Essigsäure, Maleinsäure, Bernsteinsäure, Fumarsäure, Weinsäure, Zitronensäure, Salicylsäure, Sorbinsäure, Milchsäure, sowie Sulfonsäure, wie zum Beispiel p-Toluolsulfonsäure und 1,5-Naphthalin-disulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Hauptgruppe und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure und Schwefelsäure.

Die Metallkomplexe von Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalzkkomplexe in bekannter Weise, z.B. durch Abfiltrieren, Isolieren und gegebenenfalls durch Umkristallisation reinigen.

Die erfindungsgemäss verwendbaren Wirkstoffe greifen in den Metabolismus der Pflanzen ein

und können deshalb als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab vom Zeitpunkt der Anwendung, bezogen auf das Entwicklungsstadium der Pflanzen, sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Gasschnitte in Ziergärten, Park- und Sportanlagen, an Strassenrändern, auf Flughäfen oder in Obstanlagen reduziert werden. Von Bedeutung ist auch die Hemmung des Wuchses von krautigen und holzigen Pflanzen an Strassenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (Lagerns) der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturplfanzen eine dichtere Anpflanzung, so dass Mehrerträge bezogen auf die Bodenfläche erzielt werden können. Ein Vorteil der so erzielten kleineren Pflanzen ist auch, dass die Kultur leichter bearbeitet und beerntet werden kann.

Eine Hemmung des vegetativen Wachstums der Pflanzen kann auch dadurch zu Ertragssteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, dadurch, dass mehr Assimilate gebildet werden, so dass mehr oder grössere Früchte entstehen.

Ertragssteigerungen können in manchen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zukker in Zuckerrüben, Zuckerrohr, Ananas sowie in Zitrusfrüchten zu erhöhen, oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Ausserdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper Früchte kommen. Ferner kann das Geschlecht der Blüten beeinflusst werden. Auch kann eine Sterilität des Pollens erzeugt werden, was bei der Züchtung und Herstellung von Hybridsaatgut eine grosse Bedeutung hat.

Durch den Einsatz von Wachstumsregulatoren lässt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. grosses Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine grosse Rolle, ist aber auch in anderen Kulturen, wie z.B. im Weinbau, zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden (Ausdünnung), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dies ist von besonderem Vorteil, weil sich dadurch eine optimale Anpassung an die Bedürfnisse des

Marktes herbeiführen lässt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, dass z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schliesslich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemässen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromyzetes, Oomyzetes, Chytridiomyzetes, Zygomyzetes, Ascomyzetes, Basidiomyzetes, Deuteromyzetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemässen Wirkstoffe mit besonders gutem Erfolg zur Bekämpfung solcher Pilze eingesetzt werden, die echte Mehltauerkrankungen hervorrufen, so zur Bekämpfung von *Erysiphe*-Arten, wie z.B. gegen den Erreger des Gersten- bzw. des Getreidemehltaus *(Erysiphe graminis)* und den Erreger des Gurkenmehltaus *(Erysiphe cichoreacearum)*.

Ausserdem zeigen die erfindungsgemässen Stoffe ein breites fungizides *in-vitro*-Spektrum.

In entsprechenden Konzentrationen zeigen die erfindungsgemässen Stoffe auch eine herbizide Wirkung.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweisshydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau, und organische Farbstoffe, wie Alizarin-, Azo-Metallphthalocyaninfarbstoffe, und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate angewendet werden. Die Anwendung geschieht in üblicher Weise, z.B. durch Giessen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemässen Verbindungen als Pflanzenwachstumsregulatoren können die Aufwandmengen in einem grösseren Bereich variiert werden. Im allgemeinen verwendet man pro Hektar Bodenfläche 0,01 bis 50 kg, bevorzugt 0,05 bis 10 kg.

Beim Einsatz der erfindungsgemässen Stoffe als Pflanzenwachstumsregulatoren gilt, dass die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemässen Stoffe als Fungizide kann die Aufwandmenge je nach Art der Applikation in einem grösseren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001%. Bei der Saatgutbehandlung werden im allgemeinen Wirkstoffmeng en von 0,001 bis 50 g /kg Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02%, am Wirkungsort erforderlich.

*Herstellungsbeispiele*

*Beispiel 1:*

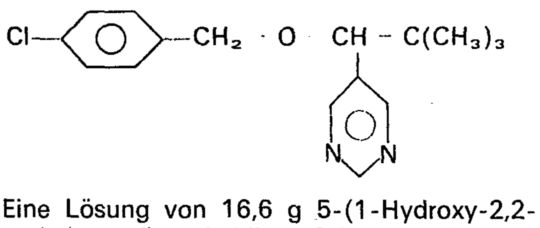

Eine Lösung von 16,6 g 5-(1-Hydroxy-2,2-dimethylpropyl)-pyrimidin, 32,2 g 4-Chlorbenzylchlorid und 6 g Tetrabutylammoniumbromid in 200 ml Toluol wird mit 200 ml 33%iger wässeriger Natronlauge versetzt. Das Reaktionsgemisch wird 18 h bei Raumtemperatur kräftig gerührt.

Die wässerige Phase wird abgetrennt, die Toluolphase mit Toluol verdünnt, viermal mit Wasser, einmal mit gesättigter wässeriger Kochsalzlösung gewaschen, über Natriumsulfat getrocknet und eingeengt. Der ölige Rückstand wird in Ether/Hexan gelöst und mit Chlorwasserstoff begast. Der entstehende kristalline Niederschlag wird abgesaugt, mit Ether nachgewaschen und mit Essigester/1n Natronlauge versetzt, wobei wieder die freie Base entsteht.

Nach Umkristallisation aus Hexan erhält man 20,3 g (70% der Theorie) 5-[1-(4-Chlorbenzyloxy)-2,2-dimethylpropyl]-pyrimidin vom Schmelzpunkt 77-78,5° C.

*Herstellung des Ausgangsproduktes*

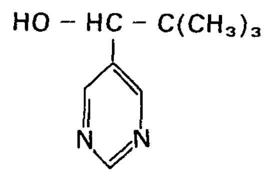
(II-1)

225 g 5-Brompyrimidin werden in 1,5 l absolutem Tetrahydrofuran/1000 ml absolutem Ether gelöst, und die Lösung wird auf −120° C abgekühlt. Bei einer Innentemperatur von −105 bis −115° C werden 250 ml 50%iges n-Butyllithium (in n-Hexan) während 2 h zugetropft. Es wird bei dieser Temperatur 1 h nachgerührt. Dann werden 309 ml Trimethylacetaldehyd innerhalb von 2 h zugetropft. Das Reaktionsgemisch wird anschliessend 4 h bei dieser Temperatur nachgerührt. Über Nacht lässt man das Reaktionsgemisch auf Raumtemperatur erwärmen und versetzt es anschliessend mit 83 g Ammoniumchlorid, gelöst in einer minimalen Menge Wasser. Die wässerige Phase wird abgetrennt, die organische Phase zweimal mit gesättigter wässeriger Kochsalzlösung gewaschen, über Natriumsulfat getrocknet und eingeengt. Nach Umkristallisation des Rückstandes aus Acetonitril erhält man 155 g (66% der Theorie) 5-(1-Hydroxy-2,2-dimethylpropyl)-pyrimidin vom Schmelzpunkt 94-96° C.

*Beispiel 2:*

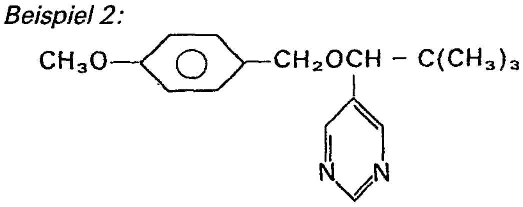

Eine Lösung von 16,6 g 5-(1-Hydroxy-2,2-dimethylpropyl)-pyrimidin in 150 ml absolutem Dimethylsulfoxid wird unter Rühren bei Raumtemperatur mit 3,3 g Natriumhydrid (80%ige in Paraffinöl) versetzt. Nach 1 h wird eine Lösung von 17,3 g 4-Methoxybenzylchlorid in 50 ml absolutem Dimethylsulfoxid zugetropft. Das Reaktionsgemisch wird anschliessend 3 Tage bei Raumtemperatur nachgerührt.

Die Dimethylsulfoxid-Lösung wird eingeengt, der Rückstand in Essigester aufgenommen, einmal mit Wasser und einmal mit gesättigter wässeriger Kochsalzlösung gewaschen, über Natriumsulfat getrocknet und eingeengt. Der Rückstand wird in Ether/Hexan gelöst und mit Chlorwasserstoff begast. Der entstehende kristalline Niederschlag wird abgesaugt, mit Ether nachgewaschen und mit Essigester/1n Natronlauge versetzt, wobei die freie Base entsteht. Man erhält 25,4 g (89% der

Theorie) 5-[1-(4-Methoxybenzyloxy)-2,2-dimethylpropyl]-pyrimidin als hellgelbes Öl mit einem Brechungsindex $n_D^{20}$ = 1,5388.

In entsprechender Weise werden die folgenden Verbindungen der allgemeinen Formel (I) erhalten:

*Tabelle 1*

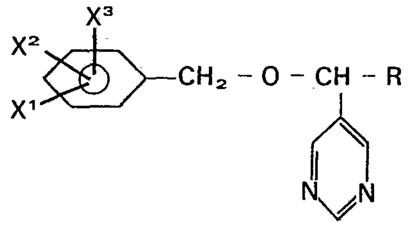

(I)

| Bsp. Nr. | $X^1$ | $X^2$ | $X^3$ | R | Schmelzpunkt (°C) bzw. Brechungsindex ($n_D^{20}$) |
|---|---|---|---|---|---|
| 3 | 2-Cl | 6-F | H | $-C(CH_3)_3$ | 63-64 |
| 4 | 4-$CH_3$ | H | H | $-C(CH_3)_3$ | 39-44 |
| 5 | 4-F | H | H | $-C(CH_3)_3$ | 1,5230 |
| 6 | 2-Cl | 4-Cl | H | $-C(CH_3)_3$ | 59-61 |
| 7 | 3-Cl | 4-Cl | H | $-C(CH_3)_3$ | 63-66 |
| 8 | 4-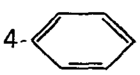 | H | H | $-C(CH_3)_3$ | 95-97 |
| 9 | 4-Cl | H | H | $-CH(CH_3)_2$ | 1,5478 |
| 10 | 4-F | H | H | $-CH(CH_3)_2$ | 1,5242 |
| 11 | 4-$CF_3$ | H | H | $-C(CH_3)_3$ | 1,4986 |
| 12 | 4-$OCF_3$ | H | H | $-C(CH_3)_3$ | 1,4949 |
| 13 | 4-$OCH_3$ | H | H | 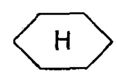 | 1,5511 |
| 14 | 4-Cl | H | H | 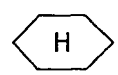 | 1,5602 |
| 15 | 4-F | H | H | 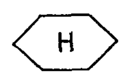 | 1,5391 |
| 16 | 3-Cl | 4-Cl | H | 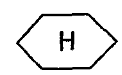 | 1,5622 |
| 17 | 2-Cl | 4-Cl | H | 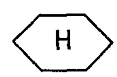 | 1,5687 |
| 18 | H | H | H | $-C(CH_3)_3$ | 1,5345 |
| 19 | 4-Cl | H | H | $-C(CH_3)_3$ | 140-142 (× HCl) |
| 20 | 4-$OCH_3$ | H | H | $-(CH_2)_4CH_3$ | 1,5320 |
| 21 | 4-Cl | H | H | $-(CH_2)_4CH_3$ | 1,5348 |
| 22 | 2-Cl | 4-Cl | H | $-(CH_2)_4CH_3$ | 1,5459 |
| 23 | 3-Cl | 4-Cl | H | $-(CH_2)_4CH_3$ | 1,5402 |
| 24 | 4-F | H | H | $-(CH_2)_4CH_3$ | 1,5155 |
| 25 | 2-Cl | 4-Cl | H | $-CH(CH_3)_2$ | 1,5592 |

Entsprechend Herstellungsbeispiel 1 werden die folgenden Ausgangsstoffe der Formel (II) erhalten:

*(Tabelle auf der nächsten Seite)*

*Anwendungsbeispiele*

In den nachfolgenden Anwendungsbeispielen werden die nachstehend angegebenen vorbekannten Verbindungen als Vergleichssubstanzen eingesetzt:

(A)

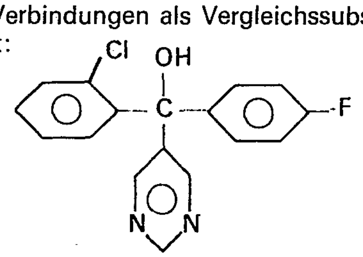

*Tabelle 2*

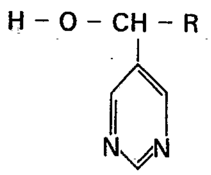

(II)

| Bsp. Nr. | R | Physikal. Konstante |
|---|---|---|
| II-2 | $-CH(CH_3)_2$ | Kp: 80-82°C/ 0,02 mbar |
| II-3 | 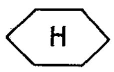 | Fp: 85-87°C |
| II-4 | 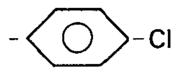 | |
| II-5 | $-(CH_2)_4-CH_3$ | Kp: 118-125°C/ 0,005 mbar |

(B) 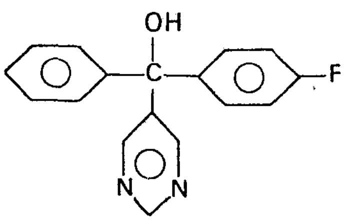

(C) 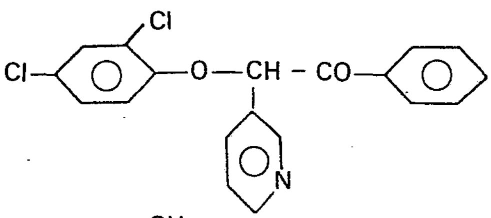

(D) 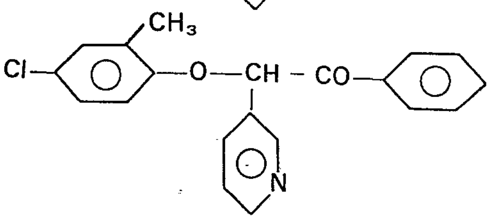

*Beispiel A:*

*Wuchshemmung bei Gras* (Festuca pratensis)

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras *(Festuca pratensis)* wird im Gewächshaus bis zu einer Wuchshöhe von 5 cm angezogen. In diesem Stadium werden die Pflanzen mit den Wirkstoffzubereitungen tropfnass besprüht. Nach 3 Wochen wird der Zuwachs gemessen und die Wuchshemmung in Prozent des Zuwachses der Kontrollpflanzen berechnet. Es bedeuten 100% Wuchshemmung den Stillstand des Wachstumes und 0% ein Wachstum entsprechend dem der Kontrollpflanzen.

Die erfindungsgemässen Wirkstoffe 1, 5, 6 und 7, 9, 10 zeigen in diesem Test eine bessere Wuchshemmung als die aus dem Stand der Technik bekannten Verbindungen (A) und (B).

*Beispiel B:*

*Wuchshemmung bei Gerste*

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstenpflanzen werden im Gewächshaus bis zum 2-Blattstadium angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 6, 18, 9, 10 zeigen in diesem Test eine bessere Wuchshemmung als die aus dem Stand der Technik bekannten Verbindungen (A) und (B).

*Beispiel C:*

*Wuchshemmung bei Baumwolle*

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit der angegebenen Menge Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 1, 3, 5, 6, 7, 8, 14, 15, 17, 9 und 10 zeigen in diesem Test eine bessere Wuchshemmung als die aus dem Stand der Technik bekannte Verbindung (A).

*Beispiel D:*

*Wuchshemmung bei Sojabohnen*

Lösungsmittel: 10 Gewichtsteile Methanol

Emulgator: 2 Gewichtsteile Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Junge Sojabohnenpflanzen werden in dem Stadium, in dem die ersten Folgeblätter entfaltet sind, mit den Wirkstoffzubereitungen tropfnass besprüht. Nach 2 Wochen wird der Zuwachs gemessen und die Wuchshemmung in Prozent des Zuwachses der Kontrollpflanzen berechnet. Es bedeuten 100% den Stillstand des Wachstums und 0% ein Wachstum entsprechend dem der Kontrollpflanzen.

Die erfindungsgemässen Wirkstoffe 1, 3, 4, 5, 6, 7, 14, 12, 11, 17, 18, 9, 10 und 2 zeigen in diesem Test eine bessere Wuchshemmung als die aus dem Stand der Technik bekannte Verbindung (A).

*Beispiel E:*

*Wuchshemmung bei Zuckerrüben*

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnass mit den Wirkstoffzubereitungen besprüht. Nach 14 Tagen wird der Zuwachs der Pflanzen gemessen und die Wuchshemmung in Prozent des Zuwachses der Kontrollpflanzen berechnet. Es bedeuten 0% Wuchshemmung ein Wachstum entsprechend dem der Kontrollpflanzen. 100% Wuchshemmung bedeutet Stillstand des Wachstums.

Die erfindungsgemässen Wirkstoffe 1, 3, 4, 5, 6, 7, 14, 12, 11, 17, 13, 18, 9, 10, 2, 8 zeigen in diesem Test eine bessere Wuchshemmung als die aus dem Stand der Technik bekannten Verbindungen (A) und (B).

*Beispiel F:*

Erysiphe-*Test (Gerste)/protektiv*

Lösungsmittel: 100 Gewichtsteile Dimethylformamid

Emulgator: 0,25 Gewichtsteile Alkyl-aryl-polyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit Sporen von *Erysiphe graminis f.sp.hordei* bestäubt.

Die Pflanzen werden im Gewächshaus bei einer Temperatur von ca. 20° C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber den aus dem Stand der Technik bekannten Verbindungen (C) und (D) zeigen bei diesem Test z.B. die Verbindungen gemäss folgender Herstellungsbeispiele: 1, 3, 4, 5, 6, 7 und 8.

*Beispiel G:*

*Steigerung der Photosynthese bei Sojabohnen*

Es wurde gefunden, dass die Verbindungen 14 und 16 bei Sojabohnen eine deutliche Steigerung der Photosynthese verursachen.

**Patentansprüche**

1. Benzyl-pyrimidinylalkyl-ether der Formel

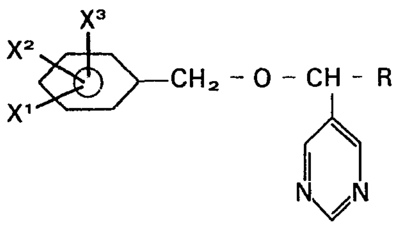

(I)

in welcher

R für Alkyl mit 1 bis 8 Kohlenstoffatomen, Cycloalkyl mit 3 bis 7 Kohlenstoffatomen oder für gegebenenfalls durch Halogen substituiertes Phenyl steht,

$X^1$ für Wasserstoff, Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen oder für Phenyl steht,

$X^2$ für Wasserstoff oder Halogen steht, und

$X^3$ für Wasserstoff oder Halogen steht,

sowie deren Säureadditionssalze und Metallsalz-Komplexe.

2. Verfahren zur Herstellung von Benzyl-pyrimidinylalkyl-ethern der Formel

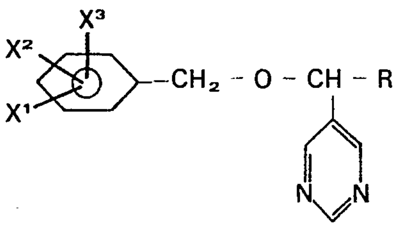

(I)

in welcher

R, $X^1$, $X^2$, $X^3$ die oben angegebene Bedeutung haben sowie von deren Säureadditionssalzen und Metallsalz-Komplexen, dadurch gekennzeichnet, dass man Pyrimidinyl-carbinole der Formel

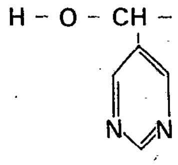 (II)

in welcher

R die oben angegebene Bedeutung hat, mit Benzylhalogeniden der Formel

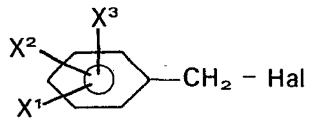 (III)

in welcher

$X^1$, $X^2$ und $X^3$ die oben angegebene Bedeutung haben und

Hal für Halogen steht,

in Gegenwart eines Lösungsmittels und gegebenenfalls in Gegenwart einer starken Base bzw. gegebenenfalls in Gegenwart eines Säurebindemittels umsetzt und gegebenenfalls eine Säure oder ein Metallsalz addiert.

3. Pflanzenwuchsregulierende und fungizide Mittel, gekennzeichnet durch einen Gehalt an mindestens einem Benzyl-pyrimidinylalkyl-ether der Formel (I) bzw. einem Säureadditionssalz oder Metallsalz-Komplex von einem Benzyl-pyrimidinylalkyl-ether der Formel (I).

4. Verfahren zur Regulierung des Pflanzenwachstums sowie zur Bekämpfung von Pilzen, dadurch gekennzeichnet, dass man Benzyl-pyrimidinylalkyl-ether der Formel (I) bzw. deren Säureadditionssalze oder Metallsalz-Komplexe auf die Pflanzen und/oder deren Lebensraum ausbringt.

5. Verwendung von Benzyl-pyrimidinylalkylethern der Formel (I) bzw. von deren Säureadditionssalzen oder Metallsalz-Komplexen zur Regulierung des Pflanzenwachstums bzw. zur Bekämpfung von Pilzen.

6. Verfahren zur Herstellung von pflanzenwuchsregulierenden und fungiziden Mitteln, dadurch gekennzeichnet, dass man Benzyl-pyrimidinylalkyl-ether der Formel (I) bzw. deren Säureadditionssalze oder Metallsalz-Komplexe mit Streckmitteln und/oder oberflächenaktiven Stoffen vermischt.

7. Pyrimidinyl-carbinole der Formel

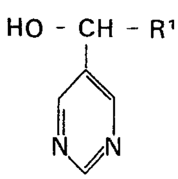 (IIa)

in welcher

$R^1$ für Isopropyl, Isobutyl, tert.-Butyl, n-Pentyl, n-Hexyl, Cyclopentyl, Cyclohexyl oder für gegebenenfalls durch Chlor substituiertes Phenyl steht.

8. Verfahren zur Herstellung von Pyrimidinylcarbinolen der Formel

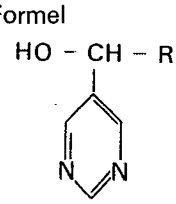 (IIa)

in welcher

$R^1$ für Isopropyl, Isobutyl, tert.-Butyl, n-Pentyl, n-Hexyl, Cyclopentyl, Cyclohexyl oder für gegebenenfalls durch Chlor substituiertes Phenyl steht, dadurch gekennzeichnet, dass man Pyrimidinhalogenide der Formel

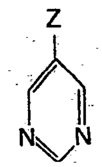 (IV)

in welcher

Z für Halogen steht, mit Aldehyden der Formel

$$O = CH-R^1 \quad (V)$$

in welcher

$R^1$ die oben angegebene Bedeutung hat, in Gegenwart eines Verdünnungsmittels und in Gegenwart einer alkalimetall-organischen Verbindung umsetzt.

9. Benzyl-pyrimidinylalkyl-ether der Formel

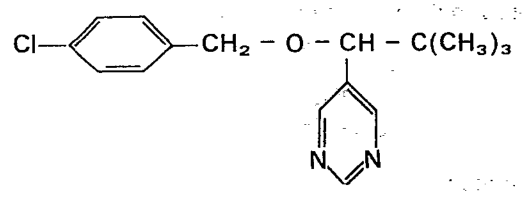

**Claims**

1. Benzyl-pyrimidinylalkyl ethers of the Formula

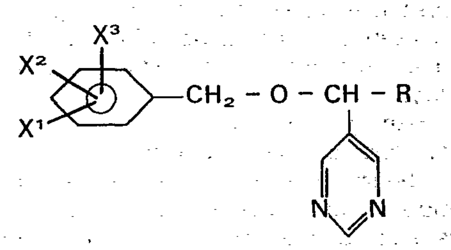 (I)

in which

R represents alkyl with 1 to 8 carbon atoms, cycloalkyl with 3 to 7 carbon atoms or phenyl which is optionally substituted by halogen,

$X^1$ represents hydrogen, halogen, alkyl with 1 to 4 carbon atoms, alkoxy with 1 to 4 carbon atoms, halogenoalkyl with 1 to 4 carbon atoms and 1 to 5 halogen atoms, halogenoalkoxy with 1 to 4 carbon atoms and 1 to 5 halogen atoms or phenyl,

$X^2$ represents hydrogen or halogen, and

$X^3$ represents hydrogen or halogen,

and acid addition salts and metal salt complexes thereof.

2. Process for the preparation of benzyl-pyrimidinylalkyl ethers of the Formula

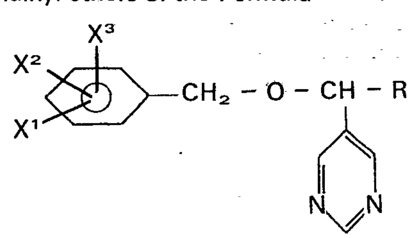 (I)

in which

R represents alkyl with 1 to 8 carbon atoms, cycloalkyl with 3 to 7 carbon atoms or phenyl which is optionally substituted by halogen,

$X^1$ represents hydrogen, halogen, alkyl with 1 to 4 carbon atoms, alkoxy with 1 to 4 carbon atoms, halogenoalkyl with 1 to 4 carbon atoms and 1 to 5 halogen atoms, halogenoalkoxy with 1 to 4 carbon atoms and 1 to 5 halogen atoms or phenyl,

$X^2$ represents hydrogen or halogen, and

$X^3$ represents hydrogen or halogen,

and of acid addition salts and metal salt complexes thereof, characterised in that pyrimidinyl-carbinols of the Formula

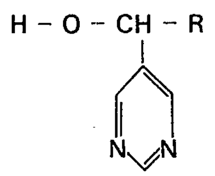

(II)

in which

R has the meaning given above, are reacted with benzyl halides of the Formula

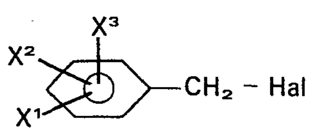

(III)

in which

$X^1$, $X^2$ and $X^3$ have the meaning given above and

Hal represents halogen,

in the presence of a solvent and, if appropriate, in the presence of a strong base or, if appropriate, in the presence of an acidbinding agent and, if required, an acid or a metal salt is added on to the product.

3. Plant-growth-regulating and fungicidal agents, characterised in that they contain at least one benzyl-pyrimidinylalkyl ether of the Formula (I) or at least one acid addition salt or metal salt complex of a benzyl-pyrimidinylalkyl ether of the Formula (I).

4. Method of regulating plant growth and of combating fungi, characterised in that benzyl-pyrimidinylalkyl ethers of the Formula (I) or acid addition salts or metal salt complexes thereof are applied to the plants and/or their habitat.

5. Use of benzyl-pyrimidinylalkyl ethers of the Formula (I) or of acid addition salts or metal salt complexes thereof for regulating plant growth or for combating fungi.

6. Process for the preparation of plant-growth-regulating and fungicidal agents, characterised in that benzyl-pyrimidinylalkyl ethers of the Formula (I) or acid addition salts or metal salt complexes thereof are mixed with extenders and/or surface-active substances.

7. Pyrimidinyl-carbinols of the Formula

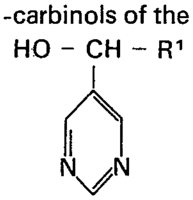

(IIa)

in which

$R^1$ represents isopropyl, isobutyl, tert.-butyl, n-pentyl, n-hexyl, cyclopentyl, cyclohexyl or phenyl which is optionally substituted by chlorine.

8. Process for the preparation of pyrimidinyl-carbinols of the Formula

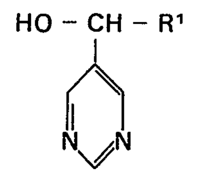

(IIa)

in which

$R^1$ represents isopropyl, isobutyl, tert.-butyl, n-pentyl, n-hexyl, cyclopentyl, cyclohexyl or phenyl which is optionally substituted by chlorine, characterised in that pyrimidine halides of the Formula

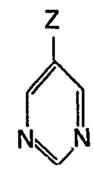

(IV)

in which

Z represents halogen, are reacted with aldehydes of the Formula

$$O = CH-R^1 \qquad (V)$$

$R^1$ has the meaning given above, in the presence of a diluent and in the presence of an alkali metalorganic compound.

9. Benzyl-pyrimidinylalkyl ethers of the Formula

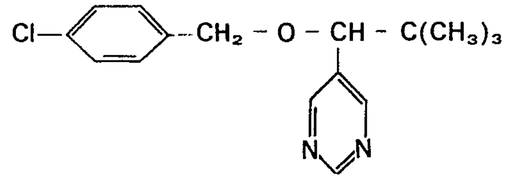

## Revendications

1. Ether-oxyde de benzyle et de pyrimidinyl-alkyle, de formule

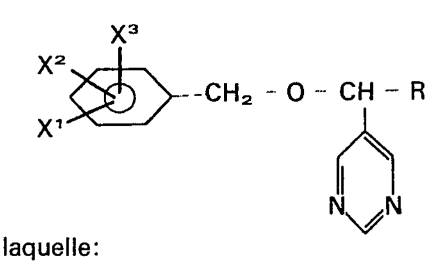

(I)

dans laquelle:

R représente un groupe alkyle ayant 1 à 8 atomes de carbone, cycloalkyle ayant 3 à 7 atomes de carbone, ou phényle éventuellement substitué par de l'halogène;

$X^1$ représente un atome d'hydrogène ou d'halogène, un groupe alkyle ayant 1 à 4 atomes de carbone, alcoxy ayant 1 à 4 atomes de carbone, halogénoalkyle ayant 1 à 4 atomes de carbone et 1

à 5 atomes d'halogène, halogénoalcoxy ayant 1 à 4 atomes de carbone et 1 à 5 atomes d'halogène, ou un groupe phényle;

$X^2$ représente un atome d'hydrogène ou d'halogène, et

$X^3$ représente un atome d'hydrogène ou d'halogène,

ainsi que leurs sels d'addition d'acides et complexes avec des sels de métaux.

2. Procédé de préparation d'éthers-oxydes de benzyles et de pyrimidinylalkyles, de formule

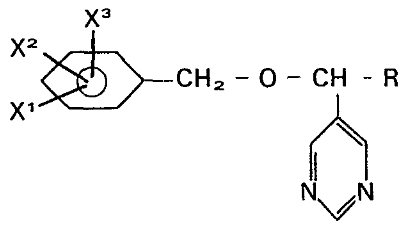 (I)

dans laquelle:

R représente un groupe alkyle ayant 1 à 8 atomes de carbone, cycloalkyle ayant 3 à 7 atomes de carbone ou un groupe phényle éventuellement substitué par de l'halogène;

$X^1$ représente un atome d'hydrogène ou d'halogène, un groupe alkyle ayant 1 à 4 atomes de carbone, alcoxy ayant 1 à 4 atomes de carbone, halogénoalkyle ayant 1 à 4 atomes de carbone et 1 à 5 atomes d'halogène, halogénoalcoxy ayant 1 à 4 atomes de carbone et 1 à 5 atomes d'halogène, ou un groupe phényle;

$X^2$ représente un atome d'hydrogène ou d'halogène, et

$X^3$ représente un atome d'hydrogène ou d'halogène, ainsi que leurs sels d'addition d'acides et complexes avec des sels de métaux, caractérisé en ce qu'on fait réagir des pyrimidinylcarbinols de formule

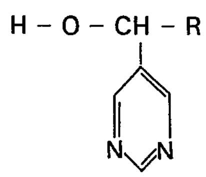 (II)

dans laquelle:

R a le sens indiqué ci-dessus

avec des halogénures de benzyles de formule:

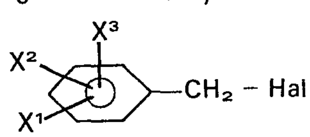 (III)

dans laquelle:

$X^1$, $X^2$ et $X^3$ ont le sens indiqué ci-dessus, et

Hal représente un halogène,

en présence d'un solvant et éventuellement en présence d'une base forte ou éventuellement en présence d'un agent de fixation des acides, et l'on fixe éventuellement par addition un acide ou un sel de métal.

3. Produit à rôle de régulation de la croissance des plantes (substance de croissance) et à rôle fongicide, caractérisé en une teneur en au moins un éther-oxyde de benzyle et de pyrimidinylalkyle de formule (I) ou d'un sel d'addition d'acide ou d'un complexe, formé avec un sel de métal, d'un éther-oxyde de benzyle et de pyrimidinylalkyle de formule (I).

4. Procédé de régulation de la croissance des plantes et pour lutter contre les champignons, procédé caractérisé en ce qu'on applique sur les plantes et/ou sur leur espace vital ou biotope des éthers-oxydes de benzyles et de pyrimidinylalkyles de formule (I), ou leurs sels d'addition d'acides ou leurs complexes avec des sels de métaux.

5. Utilisation d'éthers-oxydes de benzyles et de pyrimidinylalkyles de formule (I) ou de leurs sels d'addition d'acides ou de leurs complexes avec des sels de métaux pour la régulation de la croissance des plantes ou pour lutter contre des champignons.

6. Procédé de préparation de produits à rôle de substance de croissance et de produits fongicides, caractérisé en ce qu'on mélange des éthers-oxydes de benzyles et de pyrimidinylalkyles de formule (I) ou leurs sels d'addition d'acides ou leurs complexes avec des sels de métaux, avec des agents d'allongement et/ou des substances surfactives.

7. Pyrimidinyl-carbinols de formule:

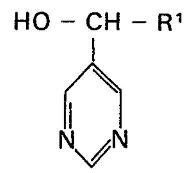 (IIa)

dans laquelle:

$R^1$ représente un groupe isopropyle, isobutyle, tertiobutyle, n-pentyle, n-hexyle, cyclopentyle, cyclohexyle ou un groupe phényle éventuellement substitué par du chlore.

8. Procédé de préparation de pyrimidinyl-carbinols de formule:

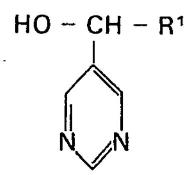 (IIa)

dans laquelle:

$R^1$ représente un groupe isopropyle, isobutyle, tertiobutyle, n-pentyle, n-hexyle, cyclopentyle, cyclohexyle, ou un groupe phényle éventuellement substitué par du chlore, procédé caractérisé en ce qu'on fait réagir des halogénures de pyrimidine de formule:

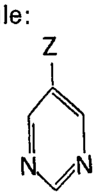 (IV)

dans laquelle:

Z représente un halogène, avec des aldéhydes de formule

$$O = CH-R^1 \quad (V)$$

dans laquelle:

$R^1$ a le sens indiqué ci-dessus, en préence d'un

diluant et en présence d'un composé organique de métal alcalin.

9. Ether-oxyde de benzyle et de pyrimidinyl-alkyle de formule: